# EUROPEAN PATENT SPECIFICATION

(11) **EP 0 313 556 B1**
(45) Date of publication and mention of the grant of the patent: **27.01.1993**
(21) Application number: 87903291.0
(22) Date of filing: 30.04.1987
(51) Int. Cl.: A61B 17/60

(54) **EXTERNAL FIXATION DEVICE**
EXTERNER FIXATEUR
DISPOSITIF DE FIXATION EXTERNE

(43) Date of publication of application: 03.05.1989
(73) Proprietor: SYNTHES AG, CH-7002 Chur (CH)
(72) Inventor: TEPIC, Slobodan, CH-7270 Davos (CH)
(74) Representative: Lusuardi, Werther Giovanni, Dr.
(86) International application number: PCT/EP87/00235
(87) International publication number: WO 88/08281

(56) References cited:
- FR-A- 1 569 090
- FR-A- 2 442 044
- US-A- 4 584 995
- Biomedizinische Technik, vol.31, no.11, November 1986 (Berlin DE) G Sipos et al: "Modellversuche zur Verbesserung der Stabilität bei einseitiger Fixateur externe Osteosynthese (Klammerfixateur); pages 261-267

## Description

### Background of the Invention

This invention relates to an external fixation device for osteosynthesis, comprising pins adapted for being passed through the bone fragments being joined, a plurality of at least two clamps for fastening said pins and at least one longitudinal rod interconnecting said individual clamps to form a single frame with the bone fragments disposed in required positions relative to each other.

Various proposals have been made for orthopaedic external fracture fixing apparatus, so-called "fixateur externes" comprising a range of components which are variably connectable with each other and with the fragments of a fractured bone by way of pins to form a support network holding the bone fragments in a desired positional relationship for the purposes of re-union.
The principle of external fixation entails load transfer from bone to an external frame by means of pins (or wires in the case of Ilizarov-type devices). Length of the pins must allow for clearing soft tissues covering the fractured bone, as well as for post-surgical treatment. The resulting distance between the treated bone and the external frame produces undesirable mechanical conditions at pin-bone interface. Parametric analyses of stress concentrations for commonly used frame configurations showed extremely high stress levels at pin-bone interface ("Parametric Analyses of Pin-Bone Stresses in External Fracture Fixation Devices" R.Huskes, E.Y.S.Chao, and T.E.Crippen, Journal of Orthopaedic Research, Vol 3, 341-349, 1985).

From the FR-A-1 569 090 a method of treatment of bone fractures is known which uses a fixation device with several clamps for receiving several pins each. The device permits a certain movement of the pin-bone extremities along a curved trajectory of unspecified and varying radius and with a centre of rotation which is located on the axis, i.e. in the center of the medullary canal of the bone to be treated. Therefore this prior art device does not allow to control the stress condition at the bone/pin interface.

None of the known external fixation devices has been able to reduce the stress concentration factors to an acceptable level. High stress levels at pin-bone interface are likely to contribute to, if not even to directly induce, pin loosening and the known clinical complications that follow. Most of the stress concentration at pin-bone interface is produced by pin angulation within cortices under functional loading as well as preloading.

### Summary of the Invention

The invention as claimed is intended to remedy these drawbacks. It solves the problem of how to design an external fixation device which couples the reaction force with the reaction moment between the pin and the rod in such a way as to keep the pin angulation at zero within bone cortices. This completely eliminates stress concentration at pin-bone interface due to bending moments on the pin.

The various features of novelty which characterize the invention are pointed out with particularity in the claims annexed to and forming part of this disclosure. For the better understanding of the invention, its operating advantages and specific objects attained by its use, reference should be had to the accompanying drawings and descriptive matter in which are illustrated and described preferred embodiments of the invention.

### Brief Description of the Drawings

In the drawings:
Fig. 1 is a schematic view of an external fixation device according to the state of the art applied to a fractured bone;
Fig. 2 is a diagrammatic view of loads and deformations in a device according to the state of the art;
Fig. 3 is a sectional view of the pin-bone interface showing stress distribution in the device according to Fig. 1;
Fig. 4 is a diagrammatic view showing conditions necessary for eliminating bending moment on the pin between bone cortices in a device according to the invention;
Fig. 5 is a sectional view showing reduced stresses at pin-bone interface in a device according to the invention;
Fig. 6 is a sectional view of a device according to the invention showing loads and deformations of the unilateral frame;
Fig. 7 is a sectional view and a cross-section of a device according to the invention showing a clamp with a gliding kinematic pair;
Fig. 8 is a sectional view of a device according to the invention showing a clamp designed as a four-bar linkage;
Fig. 9 is a sectional view of a device according to the invention showing a clamp designed as a flexible four-bar linkage;
Fig. 10 is a sectional view of a device according to the invention showing a clamp made as a composite structure;
Fig. 11 is a sectional view of a device according to the invention showing a coupled pair of clamps.

### Description of the Preferred Embodiments

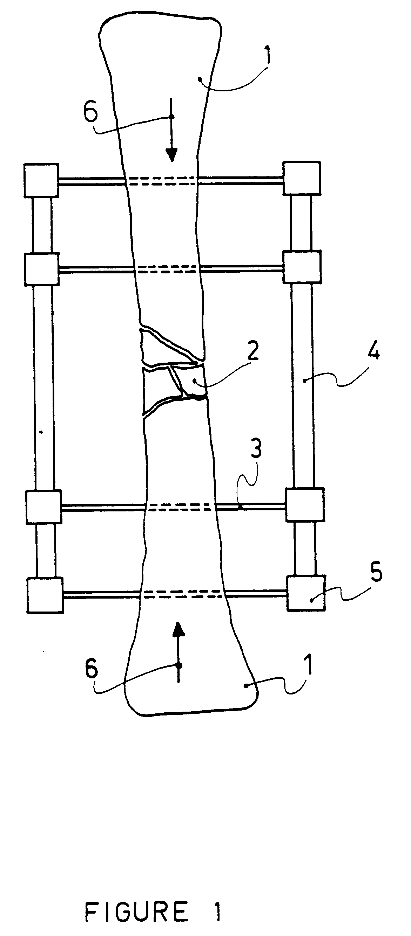

Fig. 1 represents two bone segments 1 fixed by means of an external fixation device, whereby pins 3 are inserted through the bone segments 1 and fixed to longitudinal rods 4 by means of clamps 5. Transmission of force 6 is achieved partly or completely in case of comminuted fracture 2 through external frame 3,4,5.

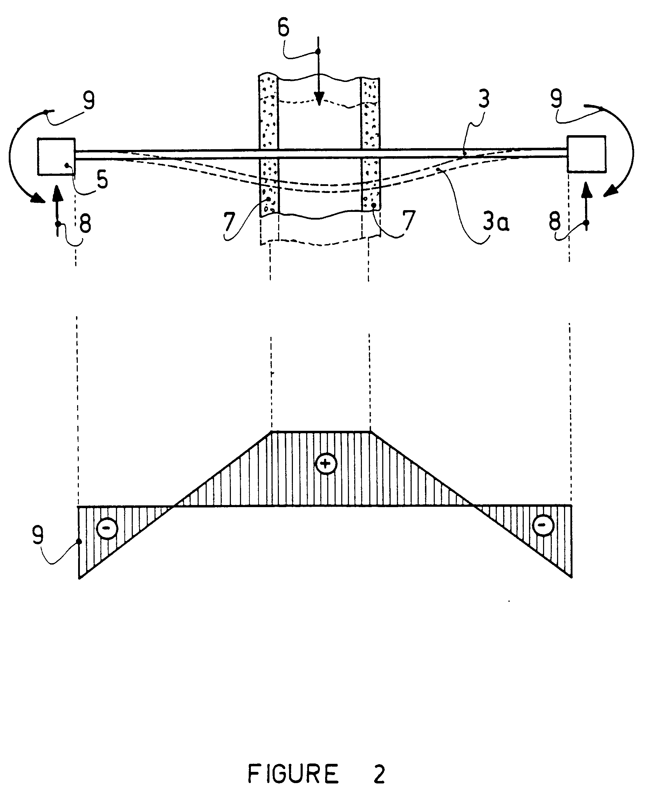

Fig. 2 shows a deformation of the pin 3 under load 6 to position 3a. Reaction forces 8 in case of symmetrical bilateral frame shown will equal one half of the axial load 6. The corresponding diagram shows bending moment of the pin 3. Reaction moments 9 depend on the pin length and bone diameter. Between bone cortices 7 bending moment is constant.

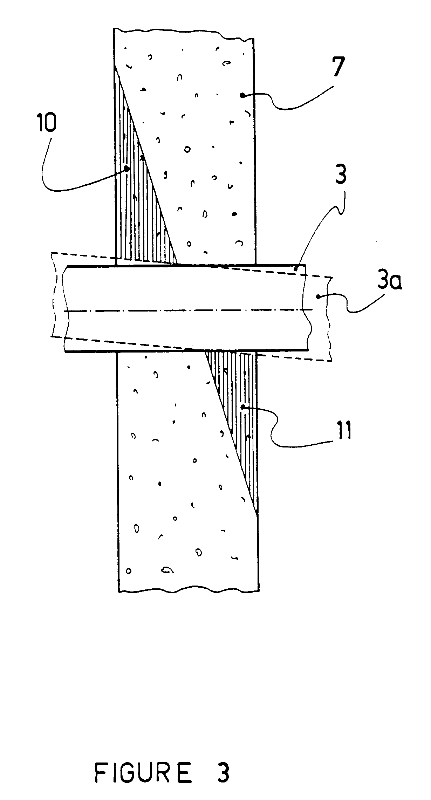

Fig. 3 shows the resultant stress distribution 10 and 11 at pin-bone interfaces due to mainly high bending moment between cortices 7.

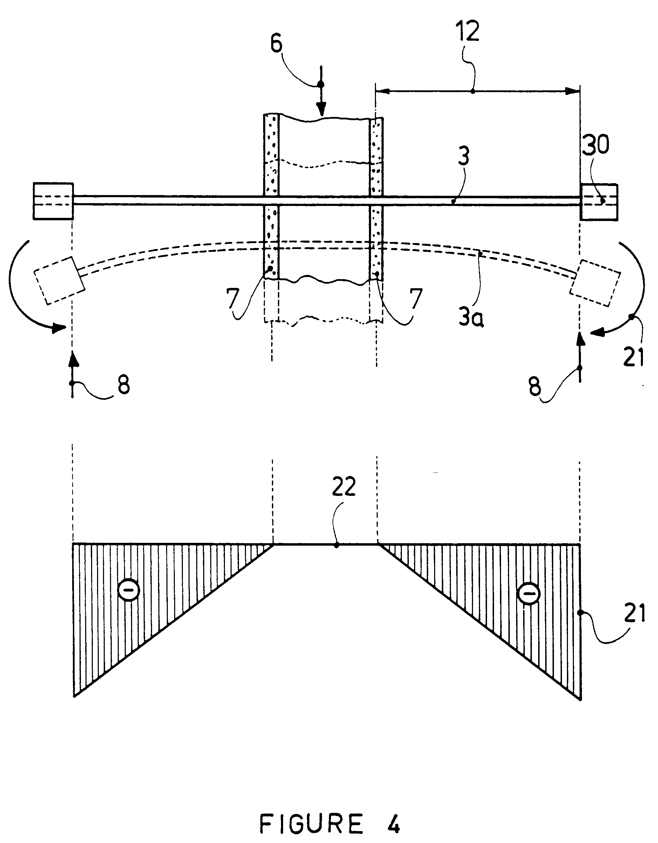

Fig. 4 shows deformations of the pin 3 into position 3a due to load 6 provided that compensating bending moments 21 are high enough to eliminate bending moment 22 between cortices 7. The magnitude of compensating bending moment 21 is equal to the product of the reaction force 8 with the free pin length 12.

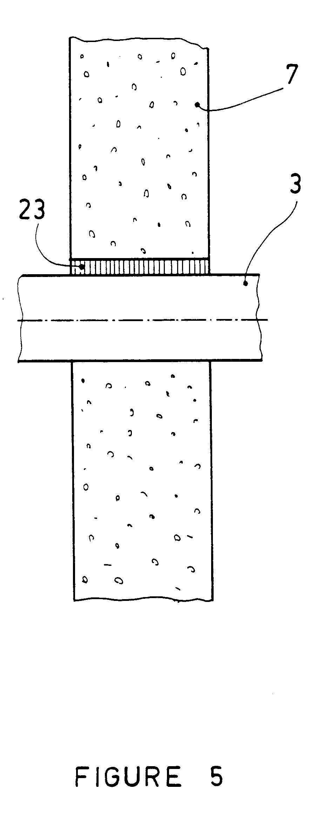

Fig. 5 shows the resultant stress distribution 23 in the device according to Fig. 4.

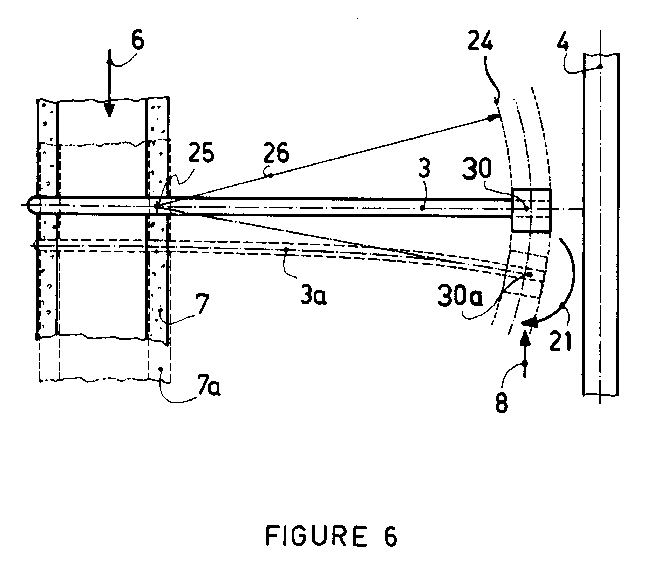

Fig. 6 shows the kinematic constraint necessary to produce conditions described by Fig. 4. If the clamped extremity 30 of the pin 3 is forced to move on a circular trajectory 24 with the center 25 within the near cortex 7 the reaction moment 21 will be equal to the product of the reaction force 8 multiplied by the radius 26 of the circle 24. Radius 26 is equal to the free pin length 12 of Fig. 4. Trajectory 24 should remain fixed with reference to the longitudinal rod 4.

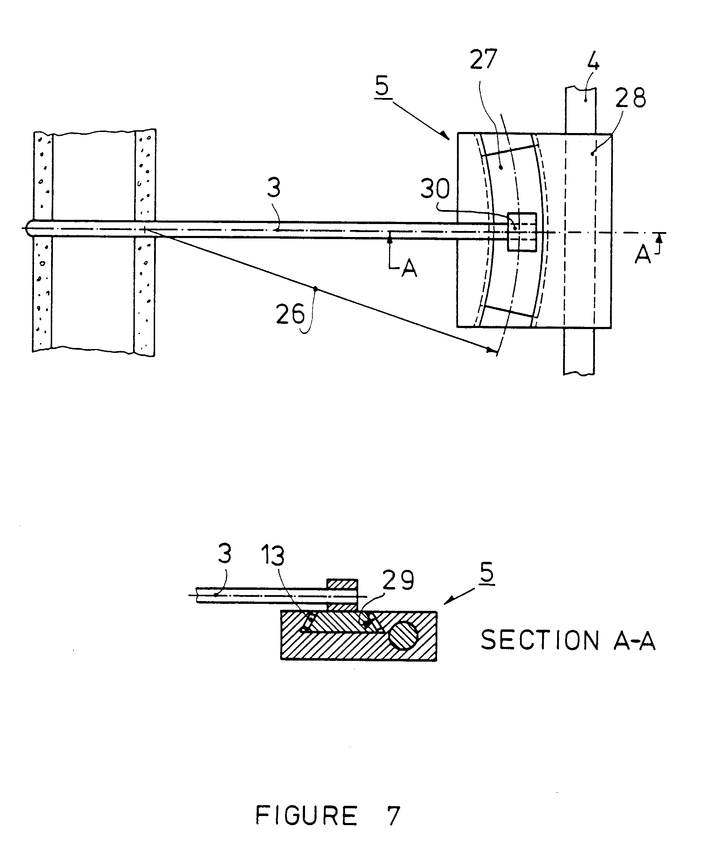

Fig. 7 shows a preferred embodiment of the clamp 5. It consists of a glider 27 which clamps pin extremity 30. Glider 27 is free to move within the circular groove 29 machined in the clamp body 28. To reduce the friction rollers 13 may be interposed between glider 27 and clamp body 28.

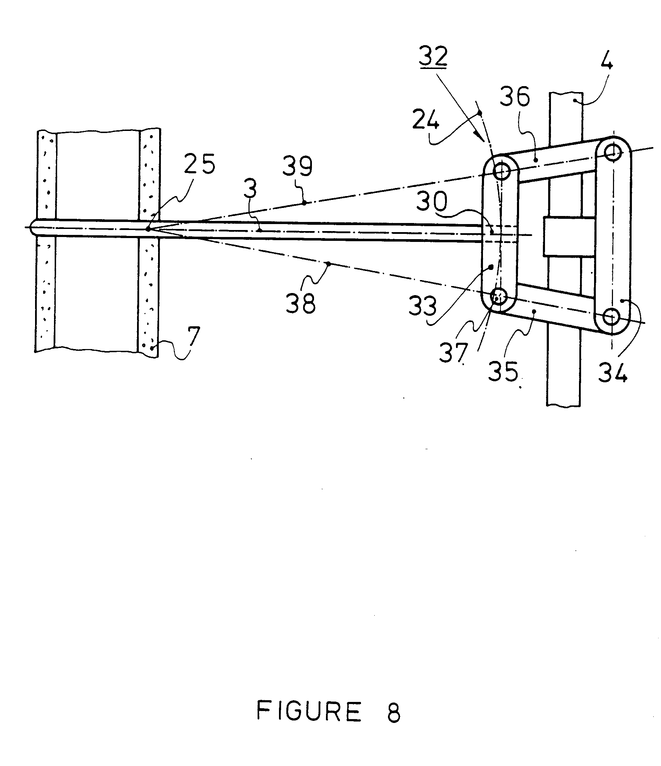

Fig. 8 shows a further preferred embodiment whereby kinematics of the pin extremity 30 is achieved by the four-bar linkage 32. The latter consists of a pin bar 33 clamping the pin 3, a rod bar 34 clamped to the longitudinal rod 4 and two side bars 35 and 36. The four bars are connected by means of joints 37. The center of rotation of the bar 33 with respect to the bar 34 lies at the intersection 25 of the axis 38 and 39 of the side bars 35 and 36. For small deformations pin extremity 30 will move on approximately circular trajectory 24.

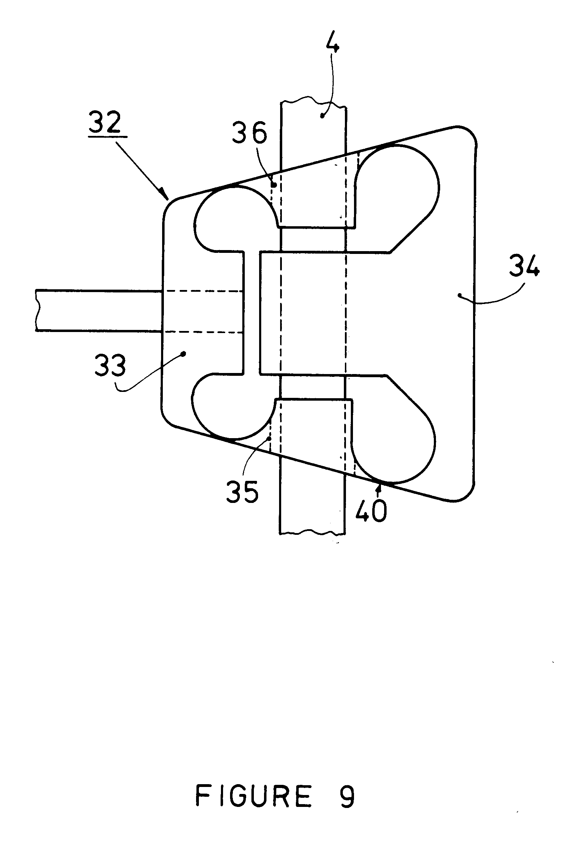

Fig. 9 shows a further preferred embodiment whereby the four-bar linkage 32 is realized by connecting the bars 33,34,35,36 by flexible sections 40. The whole four-bar linkage 32 may be manufactured from one single piece of metal. The bar 34 is fixed to the longitudinal rod 4.

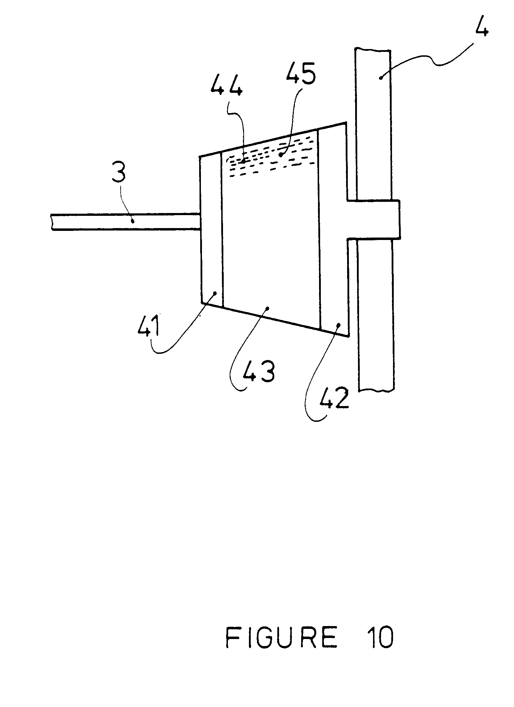

Fig. 10 shows a further preferred embodiment whereby circular kinematic trajectory 24 is realized by connecting sections 41 and 42 of clamp 5 with a composite structure 43 consisting of fibers 44 within an elastomer matrix 45.

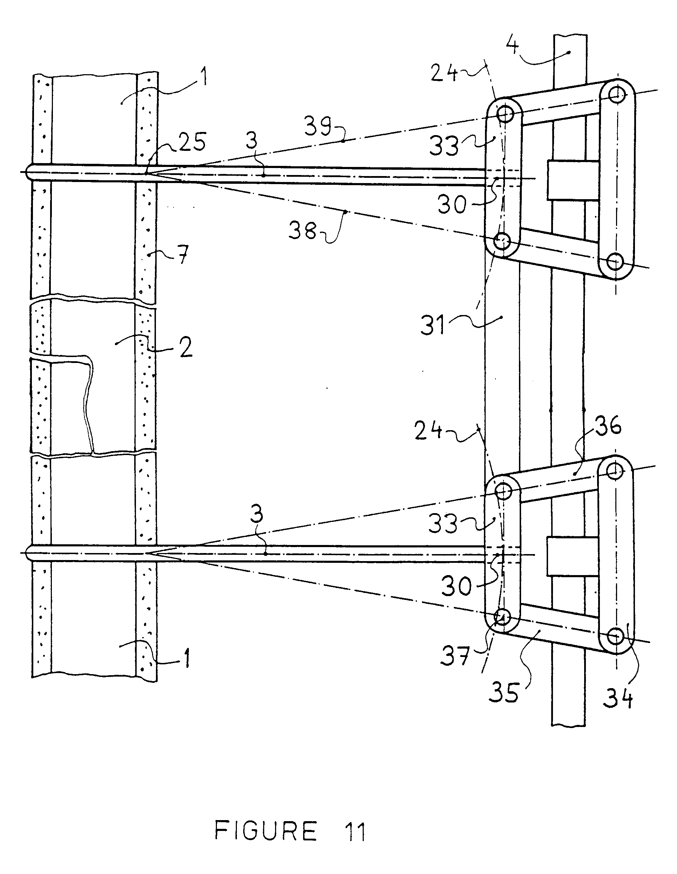

If only two pins 3 are used in each bone segment 1 an additional kinematic constraint is needed to prevent rotation of the bone segment 1 in the frame plane. This may be achieved by an additional bar linkage 31 connecting pin extremities 30 or the respective elements 33 of the clamp mechanism as shown in Fig. 11.

## Claims

1. External fixation device for osteosynthesis, comprising pins (3) adapted for being passed through the bone fragments (1) being joined, a plurality of at least two clamps (5) for fastening said pins (3), and at least one longitudinal rod (4) interconnecting said individual clamps (5) to form a single frame with the bone fragments (1) disposed in required positions relative to each other, characterised in that each said clamp (5) receives only one of said pins (3) and that it is designed in such a way that it allows movement of the axially clamped extremity (30) of said pin (3) on a trajectory (24) approximating the arc of a circle and maintaining approximately orthogonality between said clamped pin extremity (30) and said trajectory (24), whereby the center (25) of said circle can be located substantially within the near bone cortex (7) where said pin (3) enters said bone fragment (1) and the radius (26) of said circle corresponds to the free length (12) of said pin (3).

2. External fixation device according to claim 1, wherein said clamp (5) comprises a circular groove (29) suitable to receiving a glider (27) in which the extremity (30) of said pin (3) is clamped in orthogonal relationship and allowing free movement of said glider (27) within and along said circular groove (29).

3. External fixation device according to claim 1, wherein said clamp (5) comprises a four-bar linkage (32) consisting of a bar (33) in which the pin extremity (30) is clamped, of a bar (34) which is clamped to the longitudinal rod (4) and two side bars (35,36) interconnecting said two bars (33,34) by joints (37) in such a way that the axis (38,39) of said two side bars (35,36) can intersect within the near cortex (7).

4. External fixation device according to claim 3, wherein said joints (37) are replaced by flexible sections (40) forming a single framework together with said bars (33,34,35,36).

5. External fixation device according to claim 1, wherein said clamp (5) consists of an element (41) connected to said pin (3), an element (42) connected to said longitudinal rod (4) and an element (43) interconnecting said two elements (41) and (42), whereby element (43) is made of a composite material with elastomer matrix (45) and fibres (44) arranged in such a way that said element (41) is free to move on said trajectory (24).

6. External fixation device according to one of the claims 1 to 5, wherein said plurality of clamps (5) is limited to the number of two per bone fragment and said clamped pin extremities (30) of said pins (3) are connected with a bar (31) assuring synchronism to achieve rotational stability in the plane formed by said bone (1) and said longitudinal rod (4).

## Patentansprüche

1. Externe Repositionsvorrichtung zur Osteosynthese, bestehend aus entsprechenden Nadeln (3), die durch die zu verbindenden Knochenteile (1) geführt werden, sowie einer Einheit, die aus wenigstens zwei Zangen (5) zur Befestigung der Nadeln (3) und aus wenigstens einem Längsstift (4) besteht, der dieeinzelnen Zangen (5) verbindet, so daß eine einheitliche Struktur entsteht, mit deren Hilfe die Knochenteile (1) in der gewünschten Position zueinander angeordnet werden können, dadurch gekennzeichnet, daß jede einzelne Zange (5) lediglich eine der Nadeln (3) hält und daß die Zange eine Verschiebung des Endes (30) der Nadel (3) ermöglicht, wobei die senkrechte Stellung zwischen dem von einer Zange festgehaltenen Ende (30) und der kreisförmigen Kurve (24) beibehalten wird und der Mittelpunkt (25) des Kreises sich in der Masse im Innern des Markkanals (7) neben dem Knochen befinden kann, und zwar dort, wo die Nadel (3) in das Knochenstück (1) eindringt, wobei der Halbmesser (26) des Kreises der freien Länge (12) der Nadel (3) entspricht.

2. Externe Repositionsvorrichtung nach Anspruch 1, in welcher die Zange (5) eine kreisförmige Rille (29) aufweist, die eine Gleitschiene (27) aufnehmen kann, in welcher das Ende (30) der Nadel (3) in senkrechter Stellung festgehalten wird. Diese Vorrichtung ermöglicht die freie Bewegung der Gleitschiene (27) in der kreisförmigen Rille (29) und im Innern der Vorrichtung.

3. Externe Repositionsvorrichtung nach Anspruch 1, bei der die Zange (5) eine vierstäbige Verbindung (32) enthält, bestehend aus einem Stab (33), der das Ende (30) der Nadel festhält, einem Stab (34), der auf dem Längsstift (4) befestigt ist, und aus zwei seitlichen Stäben (35, 36) welche die beiden Stäbe (33, 34) durch Gelenke (37) verbinden, so daß die Achsen (38, 39) der beiden seitlichen Stäbe (35, 36) sich im Innern des naheliegenden Markkanals (7) überschneiden.

4. Externe Repositionsvorrichtung nach Anspruch 3, bei der die besagten Gelenke (37) durch flexible Teilabschnitte (40) ersetzt werden, die zusammen mit den Stäben (33, 34, 35, 36) eine einzige Struktur bilden.

5. Externe Repositionsvorrichtung nach Anspruch 1, bei der die Zange (5) aus einem mit der besagten Nadel (3) verbundenen Element (41) einem mit dem besagten Längsstift (4) verbundenen Element (42) sowie einem Element besteht, das die beiden Elemente (41) und (42) verbindet, wohingegen das Element (43) aus einem Verbundstoff mit Elastomermatrize (45) und Fasern (44) hergestellt ist, deren Anordnung die freie Bewegung des Elements (41) auf der Kurve (24) ermöglicht.

6. Externe Repositionsvorrichtung nach Ansprüche 1 bis 5, bei der die Zangeneinheit (5) sich auf zwei Zangen pro Knochenteil beschränkt, wobei die festgehaltenen Nadelenden (30) der Nadeln (3) werden durch einen Stab (31) verbunden, der den Synchronismus gewährleistet, so daß eine Rotationsstabilität auf der von dem Knochen (1) und dem Längsstift (4) gebildeten Ebene erzielt wird.

## Revendications

1. Dispositif externe de réduction pour ostéosynthèse, comprenant des aiguilles (3) adaptées pour traverser les fragments d'os (1) à relier, un ensemble d'au moins deux pinces (5) destinées à fixer lesdites aiguilles (3), et au moins une tige longitudinale (4) reliant lesdites pinces individuelles (5) de manière à former une structure unique permettant de maintenir les fragments d'os (1) en leur position mutuelle requise, caractérisé en ce que chaque dite pince (5) reçoit seulement une des dites aiguilles (3) et en ce qu'elle est conçue de manière à permettre le déplacement de l'extrémité (30) de ladite aiguille (3), pincée axialement, sur une trajectoire (24) approchant un arc de cercle, et maintenant la perpendicularité entre ladite extrémité (30) pincée de l'aiguille et ladite trajectoire (24), le centre (25) du dit cercle pouvant être situé en substance à l'intérieur du cortex (7) proche de l'os, là où ladite aiguille (3) pénètre ledit fragment d'os (1), le rayon (26) dudit cercle correspondant à la longueur libre (12) de ladite aiguille (3).

2. Dispositif externe de réduction selon la revendication 1, dans lequel ladite pince (5) comprend un sillon circulaire (29) adapté à recevoir une glissière (27) dans laquelle l'extrémité (30) de ladite aiguille (3) est pincée en position perpendiculaire, et permettant le déplacement libre de ladite glissière (27) dans ledit sillon circulaire (29) et à l'intérieur de celui-ci.

3. Dispositif externe de réduction selon la revendication 1, dans lequel ladite pince (5) comprend une liaison à quatre barreaux (32) constituée d'un barreau (33) dans lequel l'extrémité (30) de l'aiguille est pincée, d'un barreau (34) qui est pincé sur la tige longitudinale (4) et de deux barreaux latéraux (35, 36) reliant lesdits deux barreaux (33, 34) par l'intermédiaire d'articulations (37), de telle manière que les axes (38, 39) des deux dits barreaux latéraux (35, 36) puissent s'intersecter à l'intérieur du cortex proche (7).

4. Dispositif externe de réduction selon la revendication 3, dans lequel lesdites articulations (37) sont remplacées par des sections de segments flexibles (40) formant avec lesdits barreaux (33, 34, 35, 36) une structure unique.

5. Dispositif externe de réduction selon la revendication 1, dans lequel ladite pince (5) est constituée d'un élément (41) relié à ladite aiguille (3), d'un élément (42) relié à ladite tige longitudinale (4) et d'un élément (43) reliant lesdits deux éléments (41) et (42) tandis que l'élément (43) est fabriqué en un matériau composite avec une matrice élastomère (45) et des fibres (44) disposées de telle manière que ledit élément (41) soit libre de se déplacer sur ladite trajectoire (24).

6. Dispositif externe de réduction selon l'une des revendications 1 à 5, dans lequel ledit ensemble de pinces (5) est limité au nombre de deux pinces par fragment d'os, lesdites extrémités d'aiguille (30) pincées des dites aiguilles (3) étant reliées par un barreau (31) assurant un synchronisme de manière à réaliser une stabilité rotationnelle dans le plan formé par ledit os (1) et ladite tige longitudinale (4).
